## Europäisches Patentamt
## European Patent Office
## Office européen des brevets

(19)

(11) Veröffentlichungsnummer: **0 010 702**
**B1**

(12) **EUROPÄISCHE PATENTSCHRIFT**

(45) Veröffentlichungstag der Patentschrift:
05.08.81

(21) Anmeldenummer: **79104049.6**

(22) Anmeldetag: **19.10.79**

(51) Int. Cl.³: **C 07 C 69/16**, C 07 C 67/055,
C 07 C 31/20 // C07C27/02

(54) Verfahren zur Herstellung von Butendioldiacetaten und von Butandiol aus Butadien.

(30) Priorität: **28.10.78 DE 2847068**

(43) Veröffentlichungstag der Anmeldung:
**14.05.80 Patentblatt 80/10**

(45) Bekanntmachung des Hinweises auf die Patenterteilung:
**05.08.81 Patentblatt 81/31**

(84) Benannte Vertragsstaaten:
**BE DE FR GB NL**

(56) Entgegenhaltungen:
**EP 79 104 049.6
DE-A1-2 434 991
DE-A1-2 513 998
DE-A1-2 545 698
DE-A1-2 611 423
DE-A1-2 645 030
DE-A1-2 743 924
DE-B-1 191 366**

(73) Patentinhaber: **BASF Aktiengesellschaft,
Carl-Bosch-Strasse 38, D-6700 Ludwigshafen (DE)**

(72) Erfinder: **Hartig, Juergen, Dr., Dipl.-Chem., In der
Schleit 9, D-6718 Gruenstadt (DE)**
Erfinder: **Weitz, Hans-Martin, Dr.,Dipl.-Chem., Auf dem
Koeppel 40, D-6702 Bad Duerkheim (DE)**
Erfinder: **Schnabel, Rolf, Dr., Dipl.-Chem., Tilsiter
Strasse 9, D-6707 Schifferstadt (DE)**

ACTORUM AG.

## Verfahren zur Herstellung von Butendioldiacetaten und von Butandiol aus Butadien

Die Erfindung betrifft ein Verfahren zur Herstellung von Butendioldiacetat durch Umsetzung von Butadien mit Sauerstoff und einer Essigsäure in Gegenwart eines hochsiedenden Lösungsmittels an einem festen Katalysator, der ein Platinmetall und mindestens ein Element der 5. oder 6. Hauptgruppe enthält.

Es ist – z.B. aus der DT-OS 22 17 452 – bekannt, Carbonsäure-Ester eines ungesättigten Diols durch Umsetzung eines konjugierten Diens mit Sauerstoff und einer Carbonsäure an einem festen Katalysator herzustellen, der Palladium und mindestens eines der Elemente Antimon, Wismut, Tellur und Selen enthält. Nach der DT-OS 24 17 658 gelingt die Umsetzung von Butadien mit Sauerstoff und Essigsäure zu Butendioldiacetaten auch an einem festen Katalysator, der Platin und mindestens ein Element der 5. oder 6. Hauptgruppe enthält.

Die entstehenden Butendioldiacetate (BEDA) sind das cis- bzw. trans-2-Buten-1,4-dioldiacetat (1,4-BEDA) und das 1-Buten-3,4-dioldiacetat (3,4-BEDA). Sie haben Siedepunkte – bei atmosphärischem Druck – von 227,2 bzw. 234,8 bzw. 205,6 °C. Im allgemeinen überwiegen die Verbindungen, die sich vom 1,4-Diol ableiten.

Bei der technischen Durchführung dieser, als Acetoxilierungsreaktion bezeichneten Umsetzung nach bisher bekannten Verfahren sind einige Randbedingungen zu berücksichtigen, die die Wirtschaftlichkeit des technischen Verfahrens begrenzen. Hierzu gehört vor allem die Löslichkeit von Sauerstoff in Essigsäure und die erforderliche Wärmeabfuhr von etwa 196 kJ/mol BEDA (bezogen auf gasförmigen Sauerstoff, im übrigen flüssige Reaktionsteilnehmer). Zu berücksichtigen sind ferner Polymerenbildung, Einfluss des Wassers, das bei der Reaktion entsteht, Reaktionstemperatur, Katalysatorleistung usw. Aufgrund dieser Voraussetzungen enthält das erzielbare Reaktionsgemisch im allgemeinen neben etwa 1% Wasser nur 5 bis 10% BEDA und im übrigen Essigsäure. Wegen des hohen Siedepunkts der Butendioldiacetate können diese aus dem Reaktionsgemisch nur dadurch isoliert werden, dass neben dem stöchiometrisch anfallenden Wasser grosse Mengen Essigsäure abdestilliert werden. Ausserdem ist es erforderlich, die mit Wasser verdünnte Essigsäure zu entwässern, da beim fortlaufenden Verfahren die Essigsäure wieder in die Synthese zurückgeführt werden und dabei nur gewisse Wassermengen toleriert werden können. Schliesslich muss auch das abgetrennte Wasser möglichst essigsäurefrei gemacht werden. Da bekanntlich die Destillation zu den energieaufwendigsten Verfahrensmassnahmen gehört, ist die Wirtschaftlichkeit des gesamten Verfahrens in Frage gestellt. Die Erfindung hat sich die Aufgabe gestellt, ein Verfahren aufzuzeigen, das den Energiebedarf gegenüber den bekannten Verfahren deutlich herabsetzt.

Es wurde gefunden, dass man Butendioldiacetat (BEDA), speziell 1,4-BEDA und 3,4-BEDA, kostengünstig herstellen kann durch Umsetzung von Butadien mit Sauerstoff und Essigsäure an einem festen Katalysator, der ein Platinmetall und mindestens ein Element der 5. und 6. Hauptgruppe enthält, wenn man die Reaktion in Gegenwart von Butandioldiacetat, -monoacetat oder Butandiol, d.h. den Hydrierungs- und Hydrolyseprodukten des Butendiol-1,4-diacetats durchführt. Diese Verbindungen haben sämtlich Siedepunkte (bei atmosphärischem Druck) von etwa 229 °C. Die Siedepunkte des 1,2-Diols bzw. dessen Verbindungen liegen in ähnlichem Bereich.

Bei den bekannten Verfahren zur Herstellung von Butendioldiacetaten wird die Reaktion in Essigsäure durchgeführt, wobei der Essigsäure eine Doppelfunktion als Reaktionspartner und als Lösungsmittel (für Butadien und Sauerstoff) bzw. als Verdünnungsmittel zukommt. Sie wird deshalb in sehr grossem Überschuss eingesetzt und verursacht damit aber auch die bereits erwähnten hohen Energiekosten bei der Aufarbeitung.

Die erfindungsgemäss verwendeten Verbindungen, also Folgeprodukte des betrachteten Verfahrens, die im Verfahrensablauf letztlich ohnehin entstehen sind in der Lage, die Essigsäure in ihrer Lösungs- bzw. Verdünnungsfunktion zu ersetzen. Sie erweisen sich unter den Reaktionsbedingungen der Acetoxilierung als chemisch indifferent oder liefern allenfalls durch Hydrolyse Essigsäure; sie sind ausserdem mit Essigsäure mischbar.

Die Acetoxilierungsreaktion kann daher nunmehr im Prinzip mit der stöchiometrisch aus dem eingesetzten Butadien sich ergebenden Menge Essigsäure ggf. auch mit einer anderen, Essigsäure liefernden Verbindung wie Methylacetat durchgeführt werden. Ein Überschuss an Essigsäure ist natürlich möglich. Im allgemeinen wird man das Verfahren mit einem gewissen Überschuss an Essigsäure betreiben.

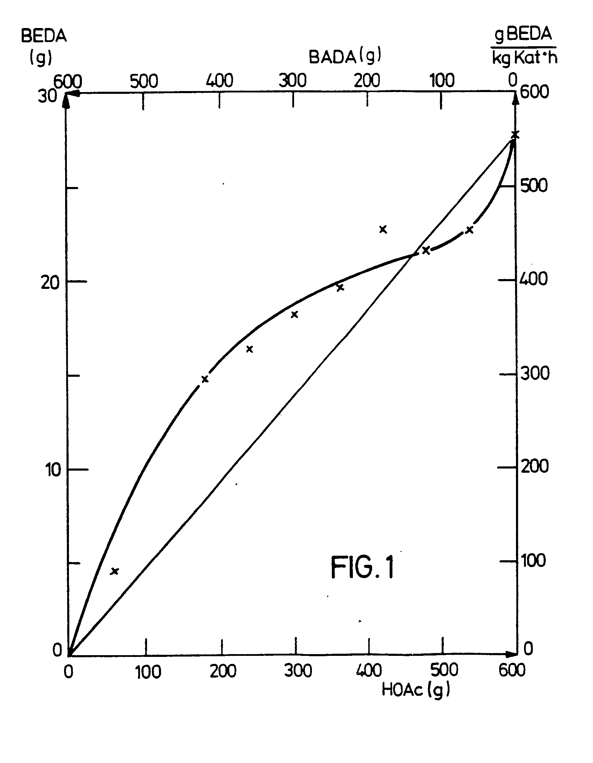

Es hat sich nämlich folgendes ergeben: Verringert man die Essigsäuremenge unter Beibehaltung der sonstigen Bedingungen, so ergibt sich zwar eine geringere Reaktionsgeschwindigkeit, jedoch ist die Reaktionsgeschwindigkeit in einem grösseren, besonders interessierenden Mischungsbereich wesentlich, d.h. überproportional höher als dem Anteil der Essigsäure im Gemisch entspricht. Ersetzt man also in einer Versuchsreihe einen Teil der ursprünglich vorhandenen Essigsäure (HOAc) systematisch durch eine entsprechende Menge von z.B. Butandioldiacetat (BADA), so erhält man die in der beigefügten Figur 1 wiedergegebene Abhängigkeit die keiner näheren Erläuterung bedarf; man sieht unmittelbar, dass die Reaktionsgeschwindigkeit (Produktivität, P) der Essigsäurekonzentration nicht proportional ist. Dies ist umso überraschender, als es normalerweise Ziel der Verfahrensentwicklung ist, ohne zusätzliche Lösungsmittel auszukommen. Inwieweit man bei einer technischen Durchführung der Acetoxilierung die Essigsäure durch eines der erfindungsgemässen Lösungsmittel ersetzt und wie man das Verhältnis Essigsäure/Butadien wählt, ist durch einen Vorversuch zu ermitteln. Prinzipiell ist jedes Verhältnis möglich. Im allgemeinen wird man pro Mol eingesetztes Butadien 0,5 bis 15, insbesondere 3 bis 9 Mol Essigsäure einsetzen. Im Lösungsmittelgemisch wird der Anteil der Verbindungen des Butandiols, also einer relativ hochsiedenden Komponente im allgemeinen zwischen 4 und 80, insbesondere zwischen 20 und 70 Gew.-%, bezogen auf flüssiges Reaktionsgemisch gewählt, liegen. In dem durch die Figur 1 veranschaulichten Fall liegt der Anteil günstig zwischen 40 und 70%.

Es kann zweckmässig sein, die Essigsäure teilweise oder vollständig durch Verbindungen zu ersetzen, welche unter den Reaktionsbedingungen Essigsäure freisetzen. Als solche Verbindungen kommen die Acetate von insbesondere einwertigen Alkoholen in Frage wie Methylacetat oder auch Butendiolmono- und/oder -diacetat, d.h. das Reaktionsprodukt selbst. Diese Ester der Essigsäure werden anstelle von Essigsäure in Gegenwart eines Hydrolyse-Katalysators und wenigstens 1 Mol Wasser pro Mol umzusetzendes Butadien verwendet. Die Hydrolyse kann vor oder während der Acetoxilierung des Butadiens erfolgen.

Nach dem erfindungsgemässen Verfahren werden nach der Acetoxilierung zunächst nicht umgesetzte oder entstandene gasförmige abzuscheidende Verbindungen entfernt (Butadien, Butene, $CO_2$ ggf. $O_2$). Ggf. kann an dieser Stelle auch die nicht umgesetzte Essigsäure und Wasser abgetrennt werden. Nach der üblichen Hydrierung (beispielsweise nach der DT-OS 25 37 890) der gebildeten Butendiolderivate wird ein Teil zurückgeführt und der Rest z.B. hydrolysiert und ggf. noch Essigsäure entfernt. Es wird dann schliesslich Butandiol oder Tetrahydrofuran als Endprodukt eines solchen Verfahrens gewonnen. In jedem Falle muss bei dem erfindungsgemässen Verfahren eine wesentlich kleinere Menge an verdünnter Essigsäure gegenüber bisher bekannten Verfahren wieder aufgearbeitet werden.

Die der Erfindung zugrundeliegende Reaktion wird in an sich bekannter Weise an einem festen Katalysator durchgeführt, der ein Platinmetall (Ruthenium, Rhodium, Palladium, Osmium, Iridium, Platin, insbesondere Palladium und/oder Platin), und mindestens ein Element der 5. oder 6. Hauptgruppe, vorzugsweise Antimon, Wismut, Schwefel, Selen und Tellur, insbesondere Tellur und Antimon, aber auch Eisenmetalle wie Nickel oder Eisen enthält. Der Katalysator ist bevorzugt ein Trägerkatalysator mit Kohlenstoff als Träger. Nach der Katalysatoranordnung kann z.B. Festbett-, Suspensions- oder Wirbelbettbetrieb gewählt werden, wobei die Reaktion absatzweise oder vorzugsweise fortlaufend durchgeführt werden kann. Die Reaktionstemperatur liegt im allgemeinen zwischen 60 und 120 °C, vorzugsweise zwischen 80 und 100 °C, Temperaturen unter 60 °C sind zwar prinzipiell möglich, jedoch sinkt der Umsatz hierbei ab. Über 120 °C nimmt die Nebenproduktbildung zu. Der Reaktionsdruck ist durch die jeweils gewählte Verfahrensanordnung gegeben und liegt im allgemeinen zwischen atmosphärischem Druck und 100 bar.

Nach dem erfindungsgemässen Verfahren entstehen Butendioldiacetate (BEDA). Als Nebenprodukte konnten gaschromatographisch in Abhängigkeit vom jeweils vorhandenen Wassergehalt gewisse Mengen an Hydrolyseprodukten (d.h. Butendiolmonoacetate bzw. Butendiole, die in der Regel ebenfalls Wertprodukte darstellen) und nur geringe Mengen nicht gewinnbarer bzw. verwertbarer Produkte nachgewiesen werden.

Bei dem Verfahren können anstelle von reinem Butadien auch butadienhaltige Kohlenwasserstoffgemische eingesetzt werden. Besonders geeignete Kohlenwasserstoffgemische sind Anteile von $C_4$-Kohlenwasserstoffen, wie sie als sog. $C_4$-Crackschnitte gewonnen werden, da das darin enthaltene Butadien erheblich kostengünstiger ist als z.B. reines Butadien.

Ein solcher $C_4$-Crackschnitt, wie er bei der Spaltung von Leichtbenzin anfällt, kann neben 1.3-Butadien ungesättigte und gesättigte Kohlenwasserstoffe, wie z.B. cis-2-Buten, trans-2-Buten, 1-Buten, iso-Buten, iso-Butan, n-Butan usw. enthalten, sollte aber, um eine Verringerung der Reaktionsgeschwindigkeit und der Katalysatorstandzeit zu vermeiden, insgesamt nicht über 0,5 Vol.-% acetylenisch ungesättigte Kohlenwasserstoffe enthalten.

Die nach den Verfahren der Erfindung herstellbaren Butendiolester, speziell Butendioldiacetate, sind wertvolle Zwischenprodukte z.B. für die Herstellung von Butandiol, Tetrahydrofuran, Adipinsäuredinitril, Hexamethylendiamin und Vitamin A.

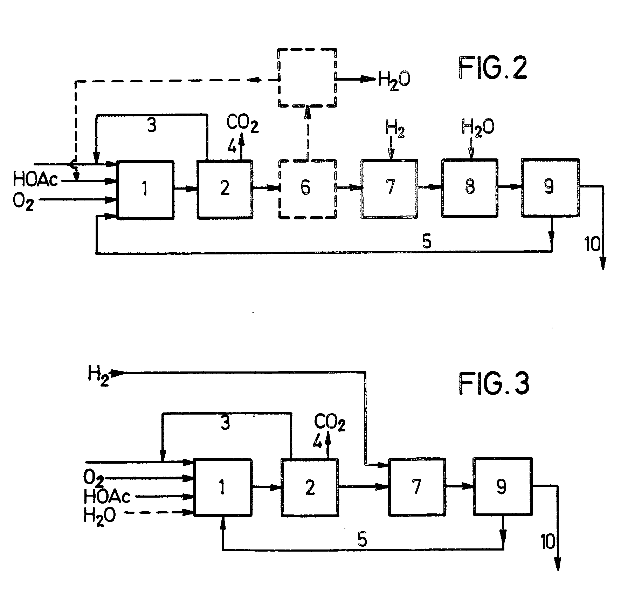

Das Verfahrensschema (Figur 2) zeigt den Gesamtprozess entsprechend vorliegender Erfindung.

In der ersten Stufe wird Butadien bzw. butadienhaltiger $C_4$-Schnitt (3) mit Essigsäure oder Essigsäure liefernden Verbindungen und Sauerstoff an geeigneten Katalysatoren in Butandioldiacetat, -monoacetat, Butandiol oder Gemischen dieser Verbindungen als Lösungsmittel (5) zu Butendioldiacetaten umgesetzt.

Danach wird zunächst das überschüssige Butadien abgetrennt (2) und zurückgeführt (3). Bei Einsatz von $C_4$-Kohlenwasserstoffgemischen erfolgt an dieser Stelle die Ausschleusung von Butenen. Kohlenoxide werden ebenfalls abgetrennt (4). Falls der Sauerstoff nicht völlig umgesetzt ist, muss auch er vor der Hydrierung entfernt werden. Gegebenenfalls kann auch die im Überschuss noch vorhandene Essigsäure, sowie das Wasser aus dem Wertprodukt abgetrennt werden (6). Dies ist zweckmässig wenn als Hydrierungskatalysatoren Nickel oder Kobalt verwendet werden. Eine Abtrennung von Essigsäure ist dagegen bei Edelmetallkatalysatoren nicht unbedingt notwendig. Nach Hydrierung (7) der gebildeten Butendiolderivate zu Butandiolderivaten wird das erhaltene Reaktionsgemisch hydrolysiert (8) und destilliert (9). Im Sumpf der anschliessenden Destillationskolonne verbleibt dann die Hauptmenge an Butandiol (10).

Eine vereinfachte Verfahrensführung zeigt Figur 3; hierbei wird die Hydrolyse zusammen mit der Synthese ausgeführt. Das Auftreten freier Essigsäure in den Produktströmen wird hierbei weitgehend vermieden.

Beispiel 1
Versuch 1 (Vergleichsversuch)

Zur Darstellung der Wirkungsweise der Erfindung werden in einen Reaktionsbehälter mit Begasungsrührer 12,5 g Katalysator, der 8,8% Palladium, 1,3% Tellur auf Aktivkohle (Körnung 0,1 bis 0,4 mm) enthält und hergestellt ist nach Beispiel 33 der DE-OS 22 17 452 (vgl. hierzu auch Beispiel 3), 600 g Essigsäure und zunächst kein Verdünnungsmittel gegeben (Versuch 1). Bei 95 °C wird ein Gemisch aus 3 Nl/h Sauerstoff und 3 Nl/h Butadien eingeleitet. Nach einer Reaktionszeit von 4 Std. bei den angegebenen Bedingungen wird die Reaktion abgebrochen, der Katalysator abgetrennt und das dabei erhaltene Rohfiltrat gaschromatographisch untersucht; ebenso wird während des Versuches jede Stunde eine Flüssigkeitsprobe gezogen. Es finden sich 27,7 g Butendioldiacetate (ungefähre Zusammensetzung: 70% trans-1,4-BEDA, 20% cis-1,4-BEDA, 10% 3,4-BEDA). (554 g BEDA/kg Katalysator.h).

Tabelle 1:
Diacetatmenge im Reaktionsgemisch (Gew.-%) bei Versuch 1 in Abhängigkeit von der Zeit

| Zeit (h) | 3,4-BEDA | cis-1,4-BEDA | tr-1,4-BEDA |
|---|---|---|---|
| 1 | 0,17 | 0,27 | 0,84 |
| 2 | 0,32 | 0,54 | 1,80 |
| 3 | 0,55 | 0,89 | 2,92 |
| 4 | 0,53 | 0,90 | 2,99 |

Versuch 2 bis 9

Es wird verfahren wie im Versuch 1 beschrieben, wobei unter Beibehaltung aller sonstigen Bedingungen die Essigsäure stufenweise durch Butandiol-1,4-diacetat der in der Tabelle 2 angegebenen Menge ersetzt wird.

In der Tabelle sind auch die Ergebnisse der Versuchsreihe zusammengestellt. In der letzten Spalte ist die «Katalysatorproduktivität» des jeweiligen Versuchs in g BEDA pro kg Katalysator und Stunde angegeben. Die Verteilung der einzelnen BEDA-Isomeren entspricht der in Tabelle 1 angegebenen. In der Figur 1 ist die BEDA-Bildung bei diesen Versuchen in Abhängigkeit von der Essigsäure- bzw. Butandioldiacetat (BEDA)-Menge graphisch aufgetragen.

Tabelle 2:

| Versuch Nr. | Essig-säure (g) | Butandiol-1,4-diacetat (g) | BEDA (g) | RZA g BEDA kg Kat.h. |
|---|---|---|---|---|
| 1 | 600 | 0 | 27.7 | 554 |
| 2 | 540 | 60 | 22.7 | 454 |
| 3 | 480 | 120 | 21.7 | 433 |
| 4 | 420 | 180 | 22.7 | 453 |
| 5 | 360 | 240 | 19.6 | 392 |
| 6 | 300 | 300 | 19.2 | 385 |
| 7 | 240 | 360 | 16.4 | 327 |
| 8 | 180 | 420 | 14.8 | 295 |
| 9 | 60 | 540 | 4.5 | 91 |

Beispiel 2

Analog Beispiel 1, Versuch 6, werden zu einem Gemisch von 300 g Butandioldiacetat und 300 g Essigsäure stündlich 3,01 l eines Gemisches aus gleichen Volumenteilen Butadien und Isobuten, unter Beibehaltung aller sonstigen Bedingungen, eingeleitet. Man erhält innerhalb von 4 Stunden insgesamt 13,8 g BEDA (RZA 277 g/kg.h). Die Anreicherung des Reaktionsgemisches an Butendioldiacetat zeigt Tabelle 3.

Tabelle 3:
Zeitabhängigkeit der Konzentration an Reaktionsprodukten im Reaktionsgemisch

| Zeit (h) | 3,4-BEDA (Gew.-%) | cis-1,4-BEDA (Gew.-%) | tr-1,4-BEDA (Gew.-%) |
|---|---|---|---|
| 0.5 | 0.05 | 0.02 | 0.34 |
| 1.0 | 0.07 | 0.08 | 0.54 |
| 1.5 | 0.11 | 0.12 | 0.76 |
| 2.0 | 0.14 | 0.15 | 0.91 |
| 2.5 | 0.18 | 0.21 | 1.16 |
| 3.0 | 0.22 | 0.24 | 1.37 |
| 3.5 | 0.24 | 0.28 | 1.49 |
| 4.0 | 0.28 | 0.34 | 1.70 |

Beispiel 3
a) Herstellung des Katalysators

Zu 350 g Aktivkohle (ein unter der Bezeichnung Desorex K handelsübliches Produkt; 0.1–0.2 mm ∅) werden 42,57 g Pd Cl$_2$ und 4,67 g TeO$_2$, gelöst in 1 Liter heisser 6-normaler Salzsäure gegeben. Das Gemisch wird unter vermindertem Druck im Rotationsverdampfer eingeengt. Der Katalysator wird bei 200 °C 4 Stunden lang unter Stickstoff getrocknet und danach 24 Stunden bei 200 °C und 24 Stunden bei 400 °C in methanolhaltigem Stickstoff reduziert. Der einsatzfähige Katalysator enthält nach Elementaranalyse 7,5% Palladium, 1,4% Tellur und 0,7% Schwefel.

b) Acetoxilierung

In einen 1 l-Schüttelautoklaven wurden folgende Komponenten eingefüllt:

  16.5 g Methylacetat
141.0 g Methanol
   4.0 g H$_2$O
207.8 g Butandiol-1,4
   5.4 g n-Butanol
   0.5 g Essigsäure
   4.5 g des o.g. Katalysators
   3.8 g Ionenaustauscher

(Lewatit S 100 H$^+$-Form, Hersteller Bayer)
20 ml flüssiges Butadien

Bei Raumtemperatur wurden 20 bar Sauerstoff eingepresst und der Autoklav 4 Stunden bei 85 °C geschüttelt. Nach dem Abfiltrieren des Katalysators wird das Gemisch unter vermindertem Druck eingeengt. Man erhält 211.5 g eines Rückstandes, der wie folgt zusammengesetzt ist:

0.08% Essigsäure
0.37% Buten-1-diol-3,4-diacetat
0.15% Buten-1-diol-3,4
0.04% Butandiol-1,4-diacetat
0.03% cis-Buten-2-diol-1,4-diacetat
2.74% tr-Buten-2-diol-1,4-diacetat
91.32% Butandiol-1,4
0.15% tr-Butendiol-1,4-monoacetat
1.36% tr-Butendiol-1,4

Die Menge an gebildeten Butendiolderivaten beträgt 10.15 g. Dieses entspricht einer Raumzeitausbeute von 564 g Produkte-/kg Katalysator.h.

## Patentansprüche

1. Verfahren zur Herstellung von Butendioldiacetat durch Umsetzung von Butadien mit Sauerstoff und Essigsäure oder einer Verbindung, die unter den Reaktionsbedingungen Essigsäure freisetzt, insbesondere an einem festen Katalysator, der ein Platinmetall und mindestens ein Element der 5. oder 6. Hauptgruppe enthält, dadurch gekennzeichnet, dass die Umsetzung in Gegenwart von Butandiol-diacetat, Butandiolmonoacetat, Butandiol oder deren Gemischen durchgeführt wird.

2. Verwendung von Butendioldiacetat, wie es gemäss Anspruch 1 erhalten wird, zur Herstellung von Butandiol-1,4 durch Hydrieren und Verseifen des Diacetats.

## Claims

1. A process for the preparation of butenediol diacetate by reacting butadiene with oxygen and acetic acid, or a compound which liberates acetic acid under the reaction conditions, in particular over a solid catalyst which contains a platinum metal and at least one element of main group 5 or 6, characterized in that the reaction is carried out in the presence of butanediol diacetate, butanediol monoacetate, butanediol or a mixture of these.

2. Use of butenediol diacetate obtained as claimed in claim 1 for the preparation of butane-1,4-diol by hydrogenating and then hydrolyzing the diacetate.

## Revendications

1. Procédé de préparation de diacétate de butènediol par réaction du butadiène avec l'oxygène et l'acide acétique, ou avec un composé libérant de l'acide acétique dans les conditions réactionnelles, en particulier en présence d'un catalyseur solide, contenant un métal du groupe du platine et au moins un élément du groupe V ou du groupe VI du Tableau périodique des Eléments, caractérisé en ce que la réaction est réalisée en présence de diacétate de butane-diol, de mono-acétate de butane-diol ou de butane-diol ou d'un mélange de ces composés.

2. Utilisation du diacétate de butène-diol, préparé par le procédé suivant la revendication 1, pour la préparation du butane-diol-1,4 par hydrogénation et saponification du diacétate.

FIG.1

FIG.2

FIG.3